Europäisches Patentamt

European Patent Office

Office européen des brevets

(19)

(11) Veröffentlichungsnummer : **0 081 751 B1**

(12) **EUROPÄISCHE PATENTSCHRIFT**

(45) Veröffentlichungstag der Patentschrift :
25.03.87

(21) Anmeldenummer : 82111131.7

(22) Anmeldetag : 02.12.82

(51) Int. Cl.⁴ : **G 01 T   1/24**, A 61 B   6/02,
**F 17 C   3/08**

(54) **Rotierendes Detektorsystem mit Kühlmittelvorrat.**

(30) Priorität : 15.12.81 DE 3149705

(43) Veröffentlichungstag der Anmeldung :
22.06.83 Patentblatt 83/25

(45) Bekanntmachung des Hinweises auf die Patenterteilung : 25.03.87 Patentblatt 87/13

(84) Benannte Vertragsstaaten :
FR NL

(56) Entgegenhaltungen :
DE-A- 2 741 404
US-A- 3 609 992
IEEE TRANS. ON NUCLEAR SCIENCE, Band NS-26,
Nr. 2, Teil II, April 1979, Seiten 2836-2839, New York,
USA D.P. BOYD: "Status of diagnostic X-ray CT"
NUCLEAR INSTRUMENTS AND METHODS IN PHY-
SICS RESEARCH, Band 188, Nr. 1, September 1981,
Seiten 63-68, Amsterdam, NL. T. MATSUZAKI et al.:
"A compton polarimeter with a pair of high-purity Ge
diodes"

(73) Patentinhaber : Siemens Aktiengesellschaft Berlin
und München
Wittelsbacherplatz 2
D-8000 München 2 (DE)

(72) Erfinder : Forster, Helmut
Forchheimer Strasse 27
D-8521 Neunkirchen/Brand (DE)

EP 0 081 751 B1

Jouve, 18, rue St-Denis, 75001 Paris, France

## Beschreibung

Die Erfindung bezieht sich auf eine Anordnung zum Herstellen eines Körperschnittsbildes, dessen Bildelemente aus der Absorption ionisierender Strahlen abgeleitet werden, die das entsprechende Körperelement in der Körperschnittebene jeweils in verschiedenen Richtungen durchsetzen, mit einem rotierenden Detektorsystem, das in der Bewegungsebene nebeneinander angeordnete Detektoren enthält, denen ein Kühlsystem zugeordnet ist. Eine derartige Anordnung ist aus der deutschen Offenlegungsschrift 2 741 404 bekannt.

Bildgebende Systeme, wie die Computer-Tomographie, mit Detektoren haben in der medizinischen Diagnostik vor allem die Aufgabe, die Anatomie des Patienten möglichst naturgetreu darzustellen. Das räumliche Auflösungsvermögen soll möglichst hoch sein, ebenso der Kontrast, mit dem sich benachbarte Organe oder Gefäße voneinander abzeichnen. Die Aufnahmezeit muß ausreichend kurz sein, um Bewegungseinflüsse auszuschalten.

Bei der Benutzung von Germanium-Detektoren, die ein besonders hohes Auflösungsvermögen haben, ist eine Kühlung mit Flüssiggas erforderlich, um das Detektorrauschen auf vernachlässigbare Werte zu vermindern. Zur Kühlung werden die Detektoren im allgemeinen auf einer Kaltfläche angeordnet, deren Rückseite wärmemäßig mit dem Kältereservoir in Verbindung steht. Die dauernde Kühlung bereitet jedoch technische Schwierigkeiten, weil die Detektorkörper während der Betriebszeit nicht nur gekühlt, sondern auch im Vakuum angeordnet sein müssen.

Aus « Nuclear Instruments and Methods in Physics Research », Bd. 188, Nr. 1, Sept. 1981, Seiten 63 bis 68 ist beispielsweise ein Compton-Polarimeter bekannt, in dem derartige Reinstgermanium-Detektoren zum Einsatz gelangen. Die Kühlung der Detektoren erfolgt dort mit Hilfe eines sogenannten « Kühlfingers », der von einem evakuierten Gehäuse vollständig umgeben ist und in den Flüssigstickstoff des Dewarbehälters hineinragt. Das gesamte, die Detektoren und den Kühlfinger umschließende evakuierte Gehäuse kann in dieser Anordnung bei ortsfesten Dewar um die Symmetrieachse des Dewargefäßes gedreht werden. Zur Herstellung eines Körperschnittbildes ist diese Anordnung nicht geeignet.

Weiterhin sind bereits stationäre (IEEE Trans. on Nuclear Science, Band NS-26, Nr. 2, Teil II, Seiten 2836 bis 2839), Detektorsysteme für den Einsatz in der Röntgen-Computer-Tomographie bekannt, in denen gekühlte Germanium-Detektoren zum Einsatz gelangen. Diese Detektoren sind über einen Tragarm an einem rechteckigen Kühlrohr befestigt, das von flüssigem Stickstoff durchströmt wird und sich im Inneren des evakuierten Detektorgehäuses befindet. Ferner sind schwenkbare Detektorsysteme bekannt (DE-A 1 27 41 404), bei denen die Detektoren jeweils in Gruppen auf einen aus Kupfer bestehenden Sockel montiert sind, der einen Teil des evakuierten Detektorgehäuses bildet und den thermischen Kontakt zum Kühlmittel des Kältebades herstellt.

Der Erfindung liegt nun die Aufgabe zugrunde, ein rotierendes Detektorsystem mit Kühlmittelvorrat zu schaffen, das keine dem Kühlmittelverbrauch angepaßte Massenausgleichsautomatik benötigt.

Die genannte Aufgabe wird nun bei einer Anordnung zum Herstellen eines Körperschnittbildes der eingangs genannten Art gelöst mit den kennzeichnenden Merkmalen des Anspruchs 1. Diese Anordnung mit den beiden Vorratsbehältern wird mit hoher Geschwindigkeit um den zu untersuchenden Gegenstand, beispielsweise einen Patienten, bewegt. Die nebeneinander in einer Reihe auf dem Kühlfinger angeordneten Detektoren können vorzugsweise mit Isolierstücken und Spannklammern auf den Kühlfinger gepreßt werden. Damit erhält man einen guten thermischen Übergang.

Das Kühlmittel, beispielsweise flüssiger Stickstoff, wird durch die Zentrifugalkraft und einen einstellbaren Regel- und Steuerdruck aus den beiden Behältern gleichmäßig entnommen und gelangt im flüssigen Zustand zum Einlaßstutzen des Detektorsystems, das den Kühlfinger enthält. Der Kühlfinger ist so bemessen, daß das Kühlmittel seine Verdampfungswärme auf die Detektoren übertragen kann, d. h., daß das Kühlmittel den Kühlfinger über den Auslaßstutzen im gasförmigen Zustand verläßt.

Zur weiteren Erläuterung wird auf die Zeichnung Bezug genommen, in der ein Ausführungsbeispiel einer Anordnung zum Herstellen eines Körperschnittsbildes mit einem Detektorsystem nach der Erfindung schematisch veranschaulicht ist.

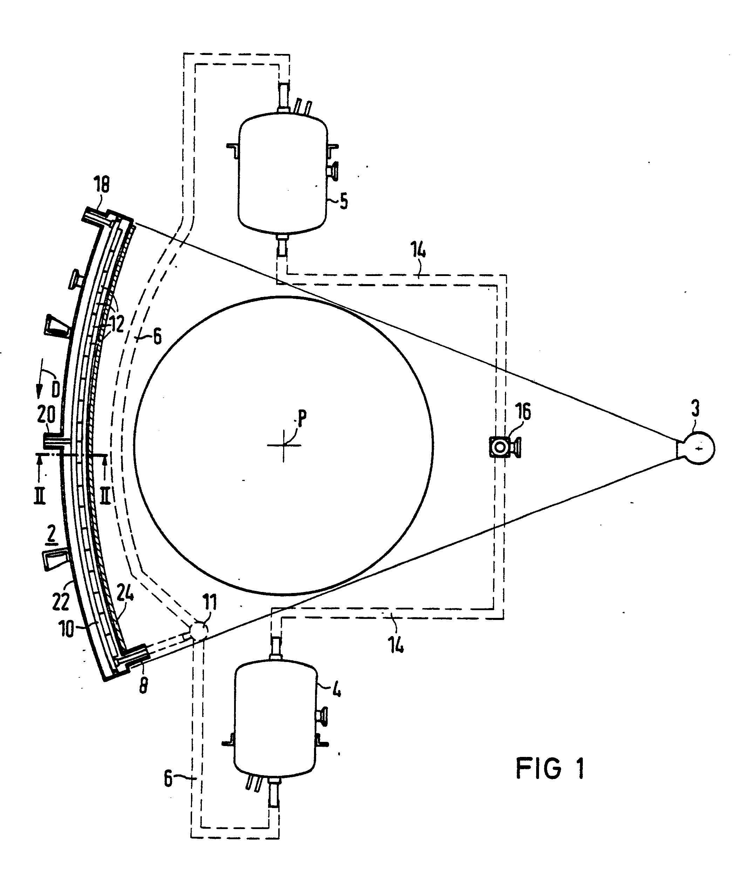

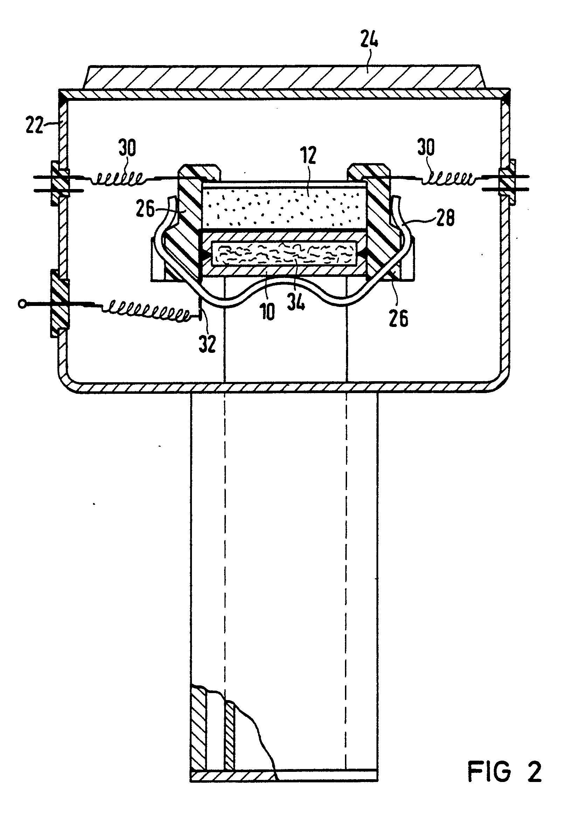

Figur 1 zeigt die gesamte Anordnung und in Figur 2 ist ein Querschnitt des Detektorsystems dargestellt.

In der Ausführungsform nach Figur 1 sind ein rotierendes Detektorsystem mit 2, zwei Kühlmittelvorratsbehälter mit 4 und 5, der Drehpunkt des Systems mit P und die Strahlungsquelle mit 3 bezeichnet, die mit Hilfe einer Blende ein fächerförmiges Strahlenbündel liefert. Von den beiden Kühlmittelvorratsbehältern 4 und 5 gelangt das Kühlmittel über eine Kryo-Leitung 6 zum Einlaßstutzen 8 eines Kühlfingers 10. Auf dem Kühlfinger 10 sind Detektoren 12 nebeneinander angeordnet. Die beiden Kühlmittelvorratsbehälter 4 und 5 sind über eine Kryo-Leitung 14 und einen Druckregler 16 miteinander verbunden. Der Kühlfinger 10 ist am Einlaßstutzen 8, einem Auslaßstutzen 18 und an einer Stütze 20 mit dem Außenmantel 22 kryotechnisch verbunden. An der dem Drehpunkt P zugewandten Seite des Außenmantels 22 ist ein Fenster 24 vorgesehen, das beispielsweise aus einem Edelstahl/Aluminium-Verbundwerkstoff bestehen kann. Bei dieser Ausführungsform gelangt das Kühlmittel, beispielsweise der flüssige Stickstoff, durch die Zentrifugalkraft aus den beiden Vorratsbehältern 4 und 5 in die Kryo-Leitung 6 und wird dann entgegen der Fliehkraft bis zum Radiusmittelpunkt 11 gedrückt. Von hier aus fließt das Kühlmittel durch die von der Umdrehungszahl abhängige Zentrifugalkraft zum Einlaßstutzen 8 des Kühlfingers 10. Das Kühlmittel wird entgegen der Drehrichtung, die in der Figur durch einen Pfeil D angedeutet ist, mit einer Kraft durch den Kühlfinger 10 gepumpt und gibt dabei seine Verdampfungswärme ab. Diese Kraft setzt sich zusammen aus mehreren Einzelkräften, nämlich der von der Drehzahl und vom Radius abhängigen Zentrifugalkraft, dem Druck in der Leitung, der durch den Regel- und Steuerdruck im Vorratsbehälter erzeugt wird, und dem Druckunterschied im Kühlfinger der abhängig ist von den Abständen des Anfangs und des Endes des Kühlfingers vom Drehpunkt P.

Die Strecke zwischen dem Kühlfingereinlaß 8 und dem -auslaß 18, auf der das Kühlmittel seinen Aggregatzustand vom flüssigen in den gasförmigen Zustand ändert, bildet einen thermischen Widerstand.

Durch diese Anordnung wird erreicht, daß das rotierende Detektorsystem mit Kühlmittelvorrat keine dem Kühlmittelverbrauch angepaßte Massenausgleichsautomatik benötigt und daß das Kühlsystem für unterschiedliche Betriebszeiten ausgelegt werden kann.

In der Ausführungsform des Kühlsystems 2 nach Figur 2 werden die Detektoren 12 durch Isolierstücke 26 und eine Spannklammer 28 auf den Kühlfinger 10 gepreßt. Zur elektrischen Kontaktierung der Detektoren 12 sind Kontaktfedern 30 vorgesehen. Außerdem ist eine Folie 32 zwischen den Detektoren 12 und dem Kühlfinger 10 angeordnet, die aus einem gut wärmeleitenden und elektrisch leitenden Material besteht und mit einem in der Figur nicht näher bezeichneten elektrischen Anschlußleiter verbunden und mit einer vakuumdichten Durchführung aus dem Außenmantel 22 herausgeführt sein kann. Der Kühlfinger 10 ist ein quaderförmiger Hohlzylinder, der vorzugsweise im Inneren mit Spänen 34 gefüllt ist, die beispielsweise aus Kupferspänen oder auch aus Edelstahlwolle bestehen können. Durch die Späne 34 im Inneren des Kühlfingers 10 wird der Wärmeaustausch zwischen Kühlmittel und den Detektoren 12 verbessert.

Diese Anordnung ist von einem Außenmantel 22 vakuumdicht umgeben, der vorzugsweise aus Edelstahl besteht und mit einem Edelstahl/Aluminium-Fenster 24 versehen sein kann. Durch diese Gestaltung erhält man einen guten thermischen Übergang zwischen den Detektoren 12 und dem Kühlfinger 10.

## Patentansprüche

1. Anordnung zum Herstellen eines Körperschnittbildes, dessen Bildelemente aus der Absorption ionisierender Strahlen abgeleitet sind, die das entsprechende Körperelement in der Körperschnittebene jeweils in verschiedenen Richtungen durchsetzen, mit einem rotierenden Detektorsystem (2) das in der Bewegungsebene nebeneinander angeordnete Detektoren (12) enthält, denen ein Kühlsystem zugeordnet ist, dadurch gekennzeichnet, daß das Kühlsystem mit einem Kühlfinger (10) versehen ist, an dem die Kühlflächen der Detektoren (12) anliegen und der von einem Kühlmittel durchströmt und über eine Kryo-Leitung (6) an zwei Kühlmittelbehältern (4, 5) angeschlossen ist, die in der Bewegungsebene des Detektorsystems (2) zentralsymmetrisch zum Drehpunkt (P) angeordnet sind.

2. Anordnung nach Anspruch 1, dadurch gekennzeichnet, daß der Kühlfinger (10) mit den Detektoren (12) im Vakuum angeordnet ist.

3. Anordnung nach Anspruch 1 mit einem Detektorsystem (2), das Detektoren (12) mit einem Halbleiterkörper aus einkristallinem Reinstgermanium enthält, dadurch gekennzeichnet, daß als Kühlmittel flüssiger Stickstoff vorgesehen ist.

4. Anordnung nach einem der Ansprüche 1 bis 3, dadurch gekennzeichnet, daß der Kühlfinger (10) als thermischer Widerstand gestaltet ist.

5. Anordnung nach einem der Ansprüche 1 bis 4, dadurch gekennzeichnet, daß die Detektoren (12) mit Hilfe von Isolierstücken (26) und Spannklammern (28) auf dem Kühlfinger (10) befestigt sind.

6. Anordnung nach einem der Ansprüche 1 bis 4, dadurch gekennzeichnet, daß die Ein- und Auslaßstutzen (8, 18) des Kühlfingers (10) kryotechnisch mit dem Außenmantel (22) des Detektorsystems (2) verbunden sind.

7. Anordnung nach Anspruch 6, dadurch gekennzeichnet, daß im Außenmantel (22) zum Drehpunkt (P) gerichtet ein Edelstahl-Aluminium-Fenster (24) vorgesehen ist.

## Claims

1. An arrangement for producing a sectional image of a body, the image elements of which are derived from the absorption of ionising beams which each pass in different directions through the corresponding body element in the sectional plane of the body, comprising a rotating detector system (2) which contains detectors (12) which are arranged one beside another in the movement plane and which are assigned a cooling system, characterised in that the cooling system is provided with a cooling finger (10) with which the cooling surfaces of the detectors (12) are in contact and which is traversed by a coolant and is connected through a coolant line (6) to two coolant containers (4, 5) which are symmetrically arranged in the plane of movement of the detector system (2) about a centre constituted by the centre of rotation (P).

2. An arrangement as claimed in Claim 1, characterised in that the cooling finger (10), together with the detectors (12), is arranged in vacuum.

3. An arrangement as claimed in Claim 1 comprising a detector system (2) which contains detectors (12) having a semiconductor body consisting of very pure monocrystalline germanium, characterised in that liquid nitrogen is provided as coolant.

4. An arrangement as claimed in one of Claims 1 to 3, characterised in that the cooling finger (10) is in the form of a thermal resistor.

5. An arrangement as claimed in one of Claims 1 to 4, characterised in that the detectors (12) are attached to the cooling finger (10) with the aid of insulating elements (26) and clamps (28).

6. An arrangement as claimed in one of Claims 1 to 4, characterised in that the inlet and outlet pipes (8, 18) of the cooling finger (10) are connected to the outer casing (22) of the detector system (2) in accordance with cryo-technique.

7. An arrangement as claimed in Claim 6, characterised in that a high grade steel-aluminium window (24) is provided in the outer casing (22) directed towards the centre of rotation (P).

**Revendications**

1. Dispositif pour réaliser une image de coupe d'un corps, dont les éléments d'image sont obtenus à partir de l'absorption de rayonnements ionisants, qui traversent l'élément correspondant du corps dans le plan de coupe de ce dernier, suivant des directions différentes, et comportant un système rotatif de détecteurs (2) qui contient des détecteurs (12) disposés côte à côte dans le plan de déplacement et auxquels est associé un système de refroidissement, caractérisé par le fait que le système de refroidissement est muni d'un doigt réfrigérant (10), contre lequel les surfaces de refroidissement du détecteur (12) sont appliquées et qui est parcouru par un fluide réfrigérant et est raccordé par l'intermédiaire d'une canalisation cryogénique (6) à deux réservoirs (4, 5) du fluide de refroidissement, qui sont disposés dans le plan de déplacement du système de détecteur (2), selon une disposition à symétrie centrale par rapport au centre de rotation (P).

2. Dispositif suivant la revendication 1, caractérisé par le fait que le doigt réfrigérant (10) est placé sous vide, ainsi que les détecteurs (12).

3. Dispositif suivant la revendication 1 comportant un système de détecteurs (2), qui contient des détecteurs (12) comportant un corps semiconducteur constitué par du germanium monocristallin extrêmement pur, caractérisé par le fait que de l'azote liquide est prévu en tant que fluide de refroidissement.

4. Dispositif suivant l'une des revendications 1 à 3, caractérisé par le fait que le doigt réfrigérant (10) est réalisé sous la forme d'une résistance thermique.

5. Dispositif suivant l'une des revendications 1 à 4, caractérisé par le fait que les détecteurs (12) sont fixés à l'aide de pièces isolantes (26) et de pinces de serrage (28) sur le doigt réfrigérant (10).

6. Dispositif suivant l'une des revendications 1 à 4, caractérisé par le fait que les tubulures d'admission et d'échappement (8, 18) du doigt réfrigérant (10) sont reliées conformément à la technique cryogénique à l'enveloppe extérieure (22) du système de détecteurs (2).

7. Dispositif suivant la revendication 6, caractérisé par le fait qu'une fenêtre (24) en acier traité-aluminium est prévue dans l'enveloppe extérieure (22), dans une disposition telle qu'elle est dirigée vers le centre de rotation (P).

**0 081 751**

FIG 1

FIG 2